# EUROPEAN PATENT APPLICATION

(11) **EP 2 532 644 A1**
(43) Date of publication of application: **12.12.2012**
(21) Application number: 12170739.2
(22) Date of filing: 04.06.2012
(51) Int. Cl.: C07C 51/363, C07C 57/30

(54) **Preparation of 2-(4-bromophenyl)-2-methylpropanoic acid**

(30) Priority: 06.06.2011 IN CH19082011
(71) Applicant: Divi's Laboratories Limited, 500 016, Hyderabad AP (IN)
(72) Inventor: Divi, Murali Krishna Prasad, 500 016 Hyperabad, Andhra Pradesh (IN); Padakandla, Gundu Rao, 500 016 Hyperabad, Andhra Pradesh (IN); Bolneni, Nageswara Rao, 500 016 Hyperabad, Andhra Pradesh (IN); Mutyala, Krishnaji Rao, 500 016 Hyperabad, Andhra Pradesh (IN)
(74) Representative: Hart-Davis, Jason

(57) **Abstract**

Selective bromination of 2-methyl-2-phenytpropanoic acid on aqueous medium is described to obtain pure 2-(4-bromophenyl)-2-methylpropanoic acid, which is a useful key intermediate in the process of manufacturing pure fexofenadine.

## Description

### Field of Invention:

The invention relates to a process for preparation of 2-(4-bromophenyl)-2-methylpropanoic acid. The 2-(4-bromophenyl)-2-methylpropanoic acid can be produced substantially free from its isomers, which is of importance to prepare fexofenadine with a high degree of purity, free from its undesirable isomers.

### Background of the Invention:

Fexofenadine is (±)-p-[1-hydroxy-4-[4-(hydroxydiphenylmethyl)piperidino]butyl]-α-methylhydratropic acid It is known as an active metabolite of antihistamine terfenadine and is marketed as a non-sedating antihistamine. Its hydrochloride salt is a pharmaceutically acceptable salt form. The USP monograph on fexofenadine hydrochloride prescribes limits for related substances and impurities. The isomeric 3-[1-hydroxy-4-[4-(hydroxydiphenylmethyl)-1-piperidinyl]butyl]-α,α-dimethyl benzeneacetic acid hydrochloride is denoted as 'fexofenadine related compound B' in the USP but is limited under 'any other unknown impurity' to less than 0.1%. On the other hand the Ph.Eur. limits this impurity specifically to not more than 0.1%. The Indian Pharmacopoeia (Ind.P.) also specifically limits this impurity to less than 0.2%. Both the Ph.Eur. and Ind.P. prescribe a separate test for determining this impurity as distinct from the test for 'Related substances'. The Pharmacopoeias also limit impurity A (which has the keto function in place of the secondary alcohol function in the side chain of fexofenadine) and the 'decarboxylated' impurity. The specified impurity B arises from the route of synthesis ordinarily followed by manufacturers as described in literature (J. Org. Chem. 1994, 59, 2620-2622). The exact source of this impurity is the key intermediate 2-(4-bromophenyl)-2-methylpropanoic acid, which may contain significant quantities of the isomeric 2-(3-bromophenyl)-2-methyl propanoic acid. This isomeric impurity in the key intermediate undergoes all the subsequent reactions in the route of synthesis and emerges as the impurity B in the fexofenadine (J. Org. Chem.*,* as cited above). The impurity A and 'decarboxylated' impurity are relatively easily removed during normal purification steps. However the impurity B, owing to its very close resemblance to the API with similar properties like polarity and solubility, is not so easily removed. Often more than three purification steps are needed to reduce this impurity to the compendial limits thus reducing yields of the API substantially and increasing cost of production. Hence it makes sense to limit this impurity at the source key intermediate. Highly pure fexofenadine can be obtained by using pure 2-(4-bromophenyl)-2-methylpropanoic acid (formula 1) as the key intermediate substantially free from the *meta* isomer (formula-2) and *ortho* isomer (formula-3).

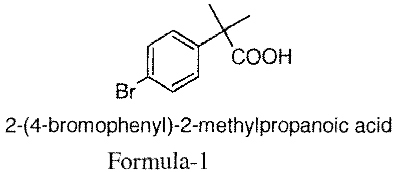

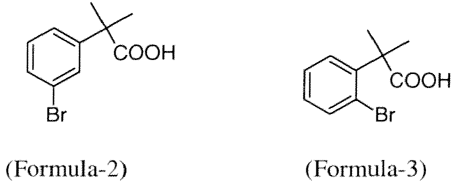

Thus, there is a need for developing an industrial process to produce pure 2-(4-bromophenyl)-2-methylpropanoic acid, which can be used as precursor to make pure fexofenadine.

### Description of prior art:

A process for preparing methyl 2-(4-bromophenyl)-2-methylpropanoate is disposed in J. Org. Chem. 1994, 59, 2620-2622. Herein, methyl 2-(4-bromophenyl)-2-methyl propanoate is prepared by esterification of 4-bromophenylacetic acid followed by methylation of the benzylic carbon employing 2.4 equivalents of methyl iodide in the presence of sodium hydride in a tetrahydrofuran medium. However, the process of selective bromination either from phenyl acetic acid or from 2-methyl-2-phenylpropanoic acid is not described. The efforts of the present inventors did not result in industrial application of this process due to the following drawbacks:
1) Preparation of 4-bromophenylacetic acid requires multistep synthesis.
2) Trimethyl chlorosilane and methyl iodide reagents are expensive to use on commercial scale.
3) Sodium hydride is a highly flammable material and an industrially hazardous reagent.
4) Tetrahydrofuran used as a solvent is also expensive to use on a commercial scale.
5) The reaction is highly exothermic.

The process for preparing 2-(4-bromophenyl)-2-methylpropanoic acid of formula-1 is also disclosed in Journal of the American Chemical Society, 1957, vol. 79, p.3432. As described therein a mixture of 2-methyl-2-phenylpropanoic acid, bromine and iron wire are refluxed in carbon tetrachloride. After workup, the product was isolated after four crystallizations. The present inventors investigated this prior art process to prepare desired compound in pure form. The results are indicated in Table-1. The present inventors were able to observe the following disadvantages:
1) The bromination reaction is non-selective, simultaneously giving rise to *meta* and *ortho* isomers of formula-2 and formula-3 respectively.
2) The bromination reaction is incomplete as indicated by the presence of unreacted starting material.
3) Isolation of pure product is not achieved due to presence of unreacted starting material and impurities.
4) Complete elimination of unreacted starting material and impurities is not achieved even after four recrystallizations.

**Table-1**

| S. No | Batch scale in grams | Yield of crude product in grams | G.C composition of crude product (% area) | | | | Yield after four crystallizations in grams | G.C composition of product After four crystallizations | | | |
|---|---|---|---|---|---|---|---|---|---|---|---|
| | | | SM | Product | Impurity | | | SM | Product | Impurity | |
| | | | | | meta | ortho | | | | meta | ortho |
| 1 | 26.6 | 30.2 | 25.14 | 62.4 | 7.70 | 1.42 | 15.1 | 11.11 | 84.94 | 2.14 | Nil |
| 2 | 26.6 | 30.1 | 35.81 | 47.26 | 5.71 | 1.10 | 10.3 | 15.78 | 65.32 | 2.10 | Nil |
| 3 | 26.6 | 30.0 | 29.73 | 56.51 | 7.59 | 1.68 | 14.6 | 12.98 | 80.23 | 2.18 | Nil |
| 4 | 26.6 | 29.9 | 25.37 | 61.74 | 7.75 | 1.75 | 15.2 | 10.78 | 86.15 | 2.99 | Nil |

| | | | | | | | | | | | |
|---|---|---|---|---|---|---|---|---|---|---|---|
| SM= Starting material: (2-methyl-2-phenylpropanoic acid); Product= 2-(4-bromophenyl)-2-methylpropanoic acid; Impurity: *meta* = 2-(3-bromophenyl)-2-methyl propanoic acid; Impurity: *ortho* = 2-(2-bromophenyl)-2-methylpropanoic acid | | | | | | | | | | | |

Thus prior art processes suffer from the following disadvantages and are not suited well for industrial application:
1) Non-selective bromination leading to formation of undesirable *meta* and *ortho* isomers (formula-2 and 3 respectively) in the key intermediate. Efforts to eliminate impurities result in low yield of pure product.
2) The reaction is incomplete requiring several recrystallizations to obtain pure product in low yield.
3) Carbon tetrachloride is a class-I solvent and toxicologically objectionable for industrial application.

For these reasons a need was felt to find a more efficient way of synthesis. The efforts of the present inventors resulted in a novel method for selective bromination of 2-methyl-2-phenylpropanoic acid to obtain 2-(4-bromophenyl)-2-methylpropanoic acid. This product can be substantially free from its isomers. Unexpectedly it was found that bromination of 2-methyl-2-phenylpropanoic acid could be achieved in an aqueous medium in acidic, neutral or alkaline conditions, avoiding undesirable halogenated hydrocarbon solvents, with excellent selectivity.

### Summary of the invention:

The present invention reveals a process for the preparation of 2-(4-bromophenyl)-2-methylpropanoic acid, which comprises reacting 2-methyl-2-phenylpropanoic acid with bromine in a water-based medium to obtain 2-(4-bromophenyl)-2-methylpropanoic acid, a useful intermediate in the process of making pure fexofenadine. The 2-(4-bromophenyl)-2-methylpropanoic acid can be obtained in a substantially pure state.

### Detailed Description of the Invention:

As used herein, 2-methyl-2-phenylpropanoic acid, a substrate for bromination, can be easily prepared from benzene, methacrylic acid and aluminium chloride as described by Prijs, B. in Helvetica Chimica Acta, vol. 35, (1952), p 780 (scheme-1)

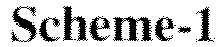

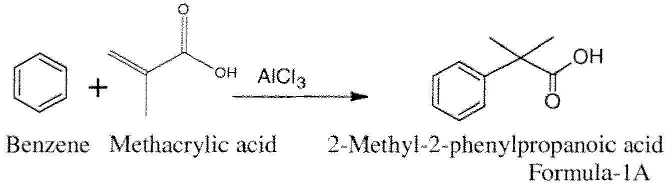

### 2-(4-Bromophenyl)-2-methylpropanoic acid was prepared by bromination of 2-methyl-2-phenytpropanoic acid in an aqueous medium as shown in scheme-2.

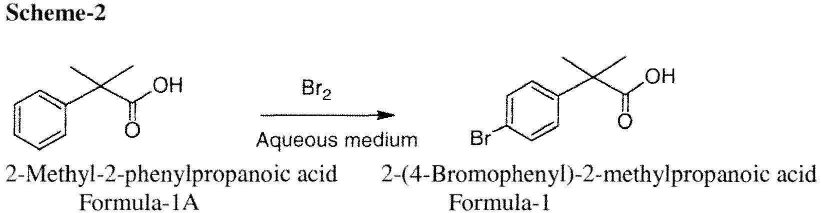

The bromination reaction can be performed in acidic, alkaline or neutral conditions. In an aqueous medium, the present inventors were surprised with the selective bromination to yield 2-(4-bromophenyl)-2-methy)propanoic acid predominantly.

The preparation of 2-(4-bromophenyl)-2-methylpropanoic acid comprises the steps of
- reacting 2-methyl-2-phenylpropanoic acid (substrate) with bromine in a water-based medium,
- extracting the heterogeneous solution with an organic solvent, and
- recovering the product.

In embodiment of the invention, the reaction is performed in heterogeneous conditions, wherein solid matter is in the reaction medium under acidic conditions. Preferably one to two equivalents of bromine to one equivalent of substrate are adequate for completion of the reactions. A large excess of bromine is not required. At less than one equivalent of bromine input, bromination was incomplete. The separation of non-brominated starting material (2-methyl-2-phenylpropanoic acid) from 2-(4-bromophenyl)-2-methylpropanoic acid product is cumbersome by crystallization due to similar solubility properties. The product mixture containing starting material can be subjected to esterification, preferably to form the methyl ester, followed by separation by means of distillation under reduced pressure. The present inventors observed no significant losses during the recovery process using the ester form, although it involves additional steps of esterification and separation by distillation.

The solvent for the extraction process can be selected from a category of water immiscible solvents wherein 2-(4-bromophenyl)-2-methylpropanoic acid is sufficiently soluble. The preferred solvents for extraction are ethers, hydrocarbons, esters, ketones, halo hydrocarbons or alcohols, most preferably dichloromethane.

The combined extracts are preferably dried and concentrated under reduced pressure to recover product as well as solvent. The practically dry residue is preferably suspended in an organic solvent in which the product is practically insoluble, such as hexanes or heptanes and filtered to recover the product. The product obtained showed about 95% content of 2-(4-bromophenyl)-2-methylpropanoic acid and about 5% content of 2-(3-bromophenyl)-2-methylpropanoic acid.

In embodiment of invention, selective bromination of 2-methyl-2-phenylpropanoic acid may be performed in a water-based medium under alkaline conditions, in which the mass is in a homogeneous condition. The product is recovered by acidifying with an acid either by filtration or by extraction with a suitable solvent. The obtained product has about 98% content of 2-(4-bromophenyl)-2-methylpropanoic acid and about 2% of 2-(3-bromophenyl)-2-methylpropanoic acid.

In another embodiment of present invention, selective bromination of 2-methyl-2-phenylpropanoic acid may be performed in a water-based medium at about pH 7 (neutral condition), wherein neutralization is achieved using alkali solution. The product is recovered by acidification followed by filtration or extraction with a suitable solvent. The product obtained has about 99% content of 2-(4-bromophenyl)-2-methylpropanoic acid and about 1% of 2-(3-bromophenyl)-2-methylpropanoic acid.

The bromination process was monitored by gas chromatographic analysis either directly by quantifying the acid form produced, or alternatively samples can be converted to the corresponding ester(s) by general method(s) of esterification using methanol and acid catalysis before analysis.

### Examples:

The following examples are for illustration purposes only and do not limit the invention in any way. The reagents and solvents mentioned in examples may be replaced by other equivalent reagents and solvents known to those skilled in the art.

### Example 1:

2-Methyl-2-phenylpropanoic acid (25 g, 0.1524 moles) and water (300 ml) were charged into a 500 ml three-necked round bottomed flask at ambient temperature (25° C to 30° C). To the resulting suspension, 43.8 g of bromine was added dropwise. The reaction mixture was stirred at 75-80°C until complete consumption of 2-methyl-2-phenylpropanoic acid as determined by gas chromatographic analysis. The reaction mixture, containing precipitated product was cooled to ambient temperature and extracted with dichloromethane (3x75 ml). The extracts were combined, dried with anhydrous sodium sulphate and evaporated to dryness. The resulting solid product was suspended in hexanes (50 ml) and filtered to recover crude product (37 g, quantitative yield), GC purity 94.4% of 2-(4-bromophenyl)-2-methylpropanoic acid and 5.5% of 2-(3-bromophenyl)-2-methylpropanoic acid. Crude product was recrystallized from aqueous methanol to give 29.0 g (78% yield) of G.C purity 99.2% 2-(4-bromophenyl)-2-methylpropanoic acid and 0.79% of 2-(3-bromophenyl)-2-methylpropanoic acid.

### Example 2:

2-Methyl-2-phenylpropanoic acid (5 g, 0.0304 moles) and sodium bicarbonate solution in water (23.06 g in 200 ml of water) were charged into a 500 ml three-necked round bottomed flask at ambient temperature (25°C to 30°C). To the resulting solution, 8.7 g of bromine was added dropwise. The reaction mixture was stirred until complete consumption of 2-methyl-2-phenylpropanoic acid as determined by gas chromatographic analysis. The reaction mixture was acidified with 5N hydrochloric acid to pH 1 to 2. The aqueous solution was extracted with dichloromethane (3x50 ml). The extracts were combined, dried with anhydrous sodium sulphate and evaporated to dryness. The resulting solid product (7.4 g, quantitative yield) was suspended in hexanes (50 ml) and filtered to recover the product (5.5 g, 74.3% yield), GC purity 98.8% of 2-(4-bromophenyl)-2-methylpropanoic acid and 1.18% of 2-(3-bromophenyl)-2-methylpropanoic acid.

### Example 3:

2-Methyl-2-phenylpropanoic acid (5 g, 0.0304 moles) and water (50 ml) were added to three-necked round bottomed flask at ambient temperature (25° C to 30° C). To the resulting mixture, sodium carbonate solution (20% in water) was added dropwise until the pH was about 7. To the resulting solution 8.7 g of bromine was added dropwise while maintaining the pH of reaction solution at about 7 by addition of sodium carbonate solution. The reaction mixture was stirred until complete consumption of 2-methyl-2-phenylpropanoic acid as determined by gas chromatographic analysis. The neutral reaction solution was acidified with 5N hydrochloric acid to pH 1 to 2. The aqueous solution was extracted with dichloromethane (3x50 ml). All organic layers were combined, dried with anhydrous sodium sulphate and evaporated to dryness. The resulting solid product was suspended in hexanes (50 ml) and filtered to recover the product, 6.0 g, 81% yield, GC purity 98.5% of 2-(4-bromophenyl)-2-methylpropanoic acid and 1.25% of 2-(3-bromophenyl)-2-methylpropanoic acid.

### Example 4:

### Step-1: Preparation of 2-methyl-2-phenylpropanoic acid:

This was prepared essentially by the method of Prijs, B (see Scheme-1 above) using benzene (5500 L) and methacrylic acid (550 Kg) in the presence of aluminium chloride (2200 Kg). The resulting product of G.C purity 98.0%, was used as such in the next step.

### Step-2: Preparation of 2-(4-bromophenyl)-2-methylpropanoic acid:

The wet material (on dry basics) from step-1 (275 Kg, assay 98%) was charged into a reactor containing 6875 L of water and 660 Kg of sodium bicarbonate. Bromine (330 Kg) was fed slowly into the reactor during 3 hours at 25-35°C and maintained for 10 hours at 25-35°C. Toluene (275 L) was charged into reactor and stirred for 15 minutes. The aqueous phase was discharged into another reactor and pH adjusted to about 5 with dilute hydrochloric acid (880 L of water and 440 L of hydrochloric acid) at 0-10°C. The resulting material was filtered and washed with water. The obtained wet product was treated with heptanes (700 L) to recover 190 Kg (46.6% yield) of 2-(4-bromophenyl)-2-methylpropanoic acid, GC purity: 99.28% of 2-(4-bromophenyl)-2-methylpropanoic acid and 0.72% of 2-(3-bromophenyl)-2-methylpropanoic acid,

### Step-3: Preparation of methyl 2-(4-bromophenyl)-2-methylpropionate:

For operational convenience the product from two or three batches of Step-2 are combined and used in this step. The wet material (on dry basis) from step-2 (575 Kg, purity of about 98.2%) was charged into a reactor containing 1150 L of toluene. The mass was maintained for about 15 minutes or until a clear solution was obtained. The toluene layer was washed with sodium chloride solution (20% in water) and separated. To the toluene layer, methanol (300 L) was charged followed by sulphuric acid addition at 25-35°C. The reaction mass temperature was gradually raised to 63-67°C and stirred for 16 hours or until complete consumption of 2-(4-bromophenyl)-2-methylpropanoic acid as indicated by GC analysis. The mass was gradually cooled to 25-35°C and then washed with water (3500 L), sodium carbonate solution (2% in water) and sodium chloride (10% solution in water) successively. The organic phase was subjected to distillation under reduced pressure to obtain methyl 2-(4-bromophenyl)-2-methylpropionate (480 Kg, 79% yield, GC purity: 99.2%).

### Step-4 Preparation of fexofenadine from methyl 2-(4-bromophenyl)-2-methylpropionate:

A mixture of 3-butyn-1-ol (30.7grams), (triphenyl phosphines) palladium (0) (1.5grams) and copper iodide (6 grams) was charged into a 1L flask containing 2-(4-bromophenyl)-2-methylpropionate (step-3 material, 75 grams, purity of about 98.2%) and potassium carbonate (120 grams) in toluene (500ml). The reaction mass temperature was gradually raised to reflux temperature and stirred for 24 hours. The resultant dark solution was filtered. The clear filtrate (toluene layer) was washed with saturated ammonium chloride solution (400ml), 20% sodium chloride solution (400ml), dried using anhydrous sodium sulphate and concentrated under reduced pressure to obtain constant weight oily material. This oily material (70 grams) was dissolved in methanol (250ml) and added to a mixture of mercuric oxide (5 grams) and 5% sulphuric acid solution in water. The reaction solution was stirred for 2 hours at 25-35°C. The reaction solution was diluted with water (500ml) and basified to pH 7-8 using ammonium hydroxide solution and extracted with ethyl acetate (2x250ml). The ethyl acetate layer was washed with water (3x350ml), dried with sodium sulphate, filtered and concentrated. The resultant brown colored thick oily material (68 grams) was treated with phosphorus oxychloride (23ml) in dichloromethane (300ml) at 25-35°C under stirring for 24 hours. The reaction was quenched by adding to chilled water (500ml). The dichloromethane layer was separated, dried with anhydrous sodium sulphate and concentrated to constant weight. The resultant oily material (48 grams) was refluxed with a mixture of azacyclonol (35 gram), potassium carbonate (94 grams) and potassium iodide (1.5gram) in toluene (250ml) for 36 hours. The reaction was quenched with chilled water (500ml) and the toluene layer separated. The aqueous layer was extracted with toluene (2x500ml). The combined toluene layers were dried and concentrated. The resulting crude material (78.4 grams) was dissolved in methanol (200 ml) at 25-35°C. To this solution, sodium borohydrate (9 grams) was added lotwise during 30 minutes (Note: addition of sodium borohydride causes an exothermic reaction). The reaction mixture was stirred for 16 hours, quenched by adding 2% acetic acid in water (20ml) and stirred for 30 minutes. The resulting solid material way filtered and dried at 60°C. The resulting solid material (31.36 grams of fexofenadine methyl ester) was dissolved in methanol (150ml). To this solution 2N sodium hydroxide solution (150ml) was added and heated to 70-75 °C for 16 hours. The reaction solution was clarified by filtration through hyflow bed and the clear filtrate was neutralized with 1N hydrochloric acid. The resulting gummy solid was stirred with methanol for 16 hours. The resulting white crystals of flexoflenadine were filtered to obtain 23.5 grams of fexofenadine invariably having a purity of 99.9% and 'impurity-B' (isomeric impurity [3-[1-hydroxy-4-[4-(hydroxydiphenylmethyl)-1-piperidinyl]butyl]-α,α-dimethyl benzeneacetic acid) below detection levels.

## Claims

1. A process for the preparation of 2-(4-bromophenyl)-2-methylpropanoic acid of formula-1 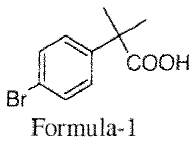 comprising bromination of 2-methyl-2-phenylpropanoic acid of formula 1A 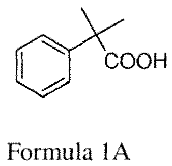 with bromine in an aqueous medium.

2. The process as in claim 1 wherein the aqueous medium is maintained in acidic or neutral or alkaline conditions.

3. The process as in claim 1 or 2 to produce 2-(4-bromophenyl)-2-methylpropanoic acid of formula-1 with a purity of greater than 98% by gas chromatographic analysis and/or less than 2% of the isomeric 2-(3-bromophenyl)-2-methylpropanoic acid.

4. Use of the product obtained from the process of any of claims 1 to 3 in a process of preparing fexofenadine with a purity of greater than 99.9% and with no detectable level of impurity B.
